# EUROPEAN PATENT SPECIFICATION

(11) **EP 2 206 480 B1**
(45) Date of publication and mention of the grant of the patent: **28.12.2011**
(21) Application number: 10150353.0
(22) Date of filing: 08.01.2010
(51) Int. Cl.: A61B 19/02

(54) **Container for sterile medical products**
Behälter für sterile medizinische Produkte
Conteneur pour produits médicaux stériles

(30) Priority: 09.01.2009 IT BO20090008
(43) Date of publication of application: 14.07.2010
(73) Proprietor: LAMPIA S.r.l., Cornate d'Adda (IT)
(72) Inventor: Grandi, Germano, 41100, Modena (IT)
(74) Representative: Lanzoni, Luciano

(56) References cited:
- EP-A2- 0 602 965
- WO-A1-2007/143863
- GB-A- 693 084
- US-A1- 2007 034 538

## Description

This invention relates to a container for sterile medical devices.

In particular, this invention relates to a container for storing and transporting devices such as, for example, filters for extracorporeal haemodialysis apparatus or the like.

Generally speaking, these devices must be transported safely from the place of their production to the place of their use. More in detail, during transportation, the device must be fully protected against structural damage. The container must therefore have exceptional shock resistance properties.

Sterile conditions must also be guaranteed during transportation so that the device concerned can be used without risks in a medical apparatus or introduced into a sterile environment such as an operating room.

Prior art containers for medical devices are box-shaped bodies in which the medical device is firmly secured to prevent shocks and/or damage.

More specifically, prior art containers comprise a separate supporting body which the device to be transported can be fastened to. One example of such kind of container is disclosed in GB693084.

The supporting body is shaped to match the medical device and is equipped with fastening means for securely holding the device to the supporting body itself.

By way of an example, straps placed round the device are removably attached to the supporting body by fastening in suitable receiving cavities forming part of clasps made as one with the supporting body.

In prior art containers the supporting body is fixed to one wall of the container itself, for example by ultrasonic welding, or by means of two-sided adhesive tape.

Disadvantageously, containers for sterile medical devices do not provide an adequate guarantee of protection against damage and maintenance of the necessary conditions of sterility. In effect, since the supporting body for the device must be firmly attached to the container wall, the presence of weld points and/or of the two-sided adhesive tape diminishes the quality of the container. More specifically, welding may cause through holes, even microscopic holes not immediately perceptible, to be made, thus making it impossible to maintain the necessary sterile conditions of the device contained.

Further, the use of two-sided adhesive tape to fix the supporting body to the container wall does not guarantee a secure hold and tends to come loose over time, leading to problems in connection with its re-use and also in connection with its disposal.

To overcome these shortcomings, a novel container, described in patent application BO2008A00064 in the name of the same Applicant as this invention, was developed.

This solution, too, however, has some shortcomings, due in particular to the difficulty of inserting the hooking element or block into the respective cavity or clip.

Indeed, precisely on account of the constructional technology of thermoformed trays, the cavities formed in the trays for the hooking elements tend to be irregular, especially when the number of cavities is relatively large compared to the size of the wall.

If the hooking elements meet excessive resistance to insertion in the cavities, the container must be rejected.

If the container is not rejected despite the resistance to insertion of the block, the operator working on the container must make an excessive effort to insert it, causing strain and leading in the long run to painful joint disorders.

Moreover, owing to the shape of the block, excessive pressure on it may cause cracks or fractures in the clip, thus breaking the sterile barrier of the container.

It is also important to note that if it is difficult to press the block into its cavity, it will be equally difficult, if not impossible, to extract it from the cavity when separating the different parts of the packaging for waste disposal and recycling.

In this context, the technical purpose which forms the basis of this invention is to propose a container for sterile medical devices which overcomes the above mentioned disadvantages of the prior art.

In particular, the aim of the present invention is to provide a container for sterile medical devices that can reduce production waste caused by the difficulty of inserting the blocks into their respective cavities.

Another aim of the invention is to provide a container for sterile medical devices that is safer against risks of damage to its sterile barrier.

The technical need indicated and the aims specified are substantially achieved by a container for sterile medical devices comprising the technical features described in one or more of the accompanying claims.

Further features and advantages of this invention are more apparent in the detailed description below, with reference to a preferred, non-limiting, embodiment of a container for sterile medical devices as illustrated in the accompanying drawings, in which:
- Figure 1 is a schematic perspective view of a container for sterile medical devices according to the invention;
- Figure 2 is a schematic perspective view, with some parts cut away in order to better illustrate others, showing a scaled-up detail of the container of Figure 1 in a first operating configuration;
- Figure 3 is a schematic perspective view of a detail from the detail of Figure 2;
- Figure 4 is a top plan view of the detail of Figure 3;
- Figure 5 is a cross section of the detail of Figure 3;
- Figure 6 is a cross section of the container of Figure 1.

With reference to the accompanying drawings, the numeral 1 denotes in its entirety a container, made in accordance with this invention, for sterile medical devices.

By way of an example, the sterile medical device might be a filtering device for extracorporeal haemodialysis apparatus or the like.

The container 1 comprises a box-shaped body 2 forming a compartment 3 for receiving at least one sterile medical device D (illustrated schematically in the drawings).

In the embodiment illustrated, the container 1 is designed to contain a single device "D". In other embodiments, not illustrated, the container 1 might be designed to contain a plurality of devices of the same type or of different types.

In the embodiment described, the box-shaped body 2 is substantially in the shape of a parallelepiped and comprises at least one substantially rectangular bottom wall 4 and four side walls 5 extending upwards from the bottom wall 4.

In embodiments that are not illustrated, the box shaped body 2 might have another shape, for example, cylindrical.

At an upper edge 6 of the side walls 5 there is a flange 7 which is substantially parallel to the bottom wall 4.

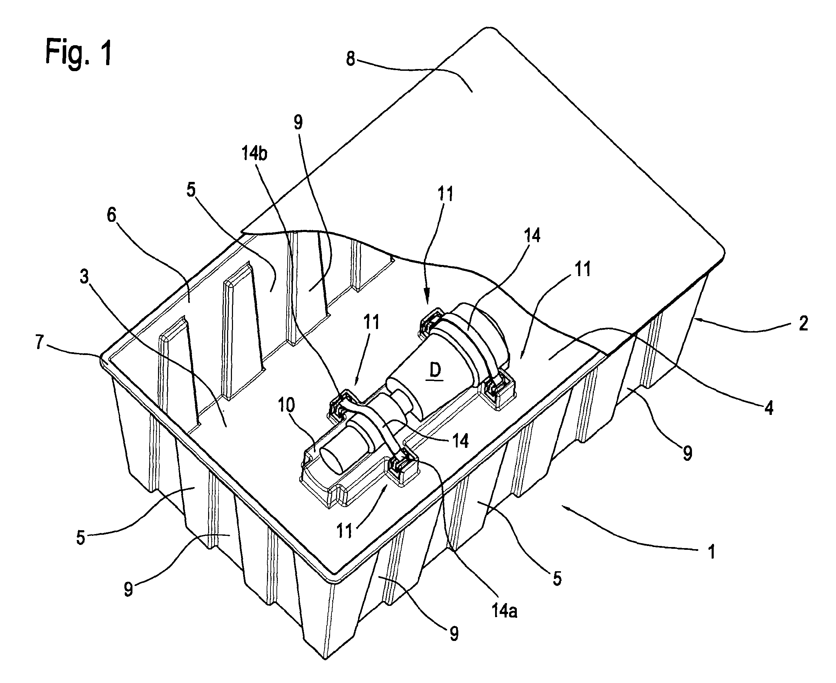

The flange 7 is necessary to couple the box-shaped body 2 to a lid 8. The lid 8 (illustrated partially in Figure 1) is in the form of a plate of plastic material fixed to the box-shaped body 2 at the flange 7 by welding, for example. The lid 8 makes it possible to maintain the necessary sterility inside the container. To open the container 1 when the device "D" is needed, it is sufficient to take the lid 8 off the box-shaped body 2 to gain access to the containment compartment 4.

The side walls 5 have a plurality of grooves or ribs 9 extending from the bottom wall 4 towards the flange 7. The ribs 9 are designed to stiffen the box-shaped body 2. In effect, the latter must have considerable shock resistance properties so that it can fully protect the device "D" it contains against structural damage.

The bottom wall 4 of the box-shaped body 2 comprises a portion 10 for receiving the device "D". The receiving portion 10 is a portion of the bottom wall 4 shaped to match the device "D" so that the latter can be fitted snugly in the containment compartment 3.

It should be noted that the receiving portion 10 is made as one with the rest of the bottom wall 4, for example by vacuum thermoforming.

The container 1 also comprises fastening means 11 connected to the box-shaped body 2 in order to removably secure the medical device "D" to the box-shaped body 2 itself.

The fastening means 11 comprise at least one hooking element 12 connected to the device "D" and a matching receiving cavity 13 for the hooking element 12 where the latter is housed in order to secure the device "D" to the box-shaped body 2.

In the embodiment described, the fastening means 11 comprise two pairs of hooking elements 12 and two matching pairs of receiving cavities 13.

In alternative embodiments not illustrated, the fastening means 11 comprise a plurality of hooking elements 12.

According to this invention, at least one receiving cavity 13 is made as one with the bottom wall 4 of the box-shaped body 2. Preferably, all the receiving cavities 13 are made as one with the bottom wall 4. More in detail, the receiving cavities 13 are made by vacuum thermoforming of the bottom wall 4 itself.

The fastening means 11 also comprise at least one strap 14 whose ends 14a, 14b are connected to two respective hooking elements 12.

More specifically, each hooking element 12, which is described in more detail below, comprises a slot 18 used to connect the strap 14 to the hooking elements 12.

More in detail, in the embodiment described, the fastening means 11 comprise two straps 14, each associated with a respective pair of hooking elements 12.

Each strap 14 is thus fastened to the box-shaped body 2 through the hooking elements 12 inserted in the receiving cavities 13. Further, each strap 14 is placed over the device "D" in such a way as to press the device "D" itself against the bottom wall 4 and, more specifically, against the receiving portion 10 of the bottom wall 4.

Each strap 14 is adjustable in length so that the strap 14 can be adapted to the device "D".

More specifically, each strap 14 has a first end 14a secured to the slot 18 at a respective hooking element 12 and a second end 14b slidably associated with another hooking element 12. In detail, the second end 14b of the strap 14 is slidably inserted into the slot 18 of the respective hooking element 12.

Further, the strap 14 has a folded flap 14c which extends from the second end 14b and is folded back over the rest of the strap 14 and attached to the latter for example by a piece of Velcro. Thus, by acting on the folded flap 14c, the length of the strap 14 can be adjusted in order to secure the device "D" to the box-shaped body 2.

Each of the receiving cavities 13 is formed by two tabs 15 connected to the bottom wall 4 and extending towards each other.

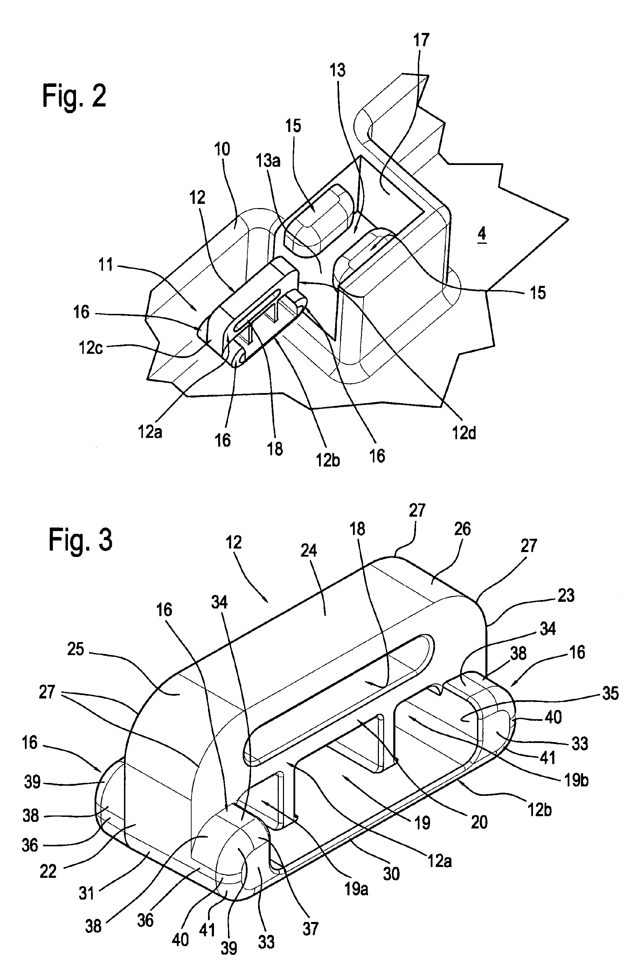

Looking in more detail at the hooking elements 12 with reference to Figures 2 to 6, it may be observed that the sides of the hooking elements 12 are preferably joined to each other by curved surfaces.

In other words, the edges of the hooking elements, in the sense of intersecting lines where two or more of the surfaces of the hooking elements meet, are rounded.

The hooking elements 12 can be inserted by sliding them in the respective receiving cavities 13 so that they lie, at least partly, between the tabs 15.

When the hooking element 12 is inserted into the respective cavity 13, the first portion 12a lies completely between the tabs 15 while the second portion 12b lies substantially under the tabs 15. With reference to Figure 2, when each hooking element 12 is slidably inserted into the respective receiving cavity 13, it lies on a supporting surface 13a of the receiving cavity 13 and is pushed against a stop wall 17. When the corresponding strap 14 is tightened on the medical device "D", the strap 14 raises the hooking elements 12 located at their ends 14a, 14b.

More in detail, each hooking element 12 comprises a substantially flat, first portion 12a and a substantially flat, second portion 12b connected transversally as one with the first portion 12a. The first flat portion 12a comprises the slot 18 and, below that, with reference to Figure 3, a lightening opening or undercut 19.

Advantageously, the portion 12a has two further openings 19a and 19b located at the sides of the opening 19 in order to further lighten the element 12 and to save on the material needed to make it. As shown in particular in Figure 3, the portion 12a has a first face 20, a second face 21 parallel to the first face 20, a third face 22 transversal to the first and second faces 20, 21 and a fourth face 23 parallel to the third face 22.

Looking at Figure 3, the portion 12a also has a top face 24 transversal to the first and second faces 20, 21 and to the third and fourth faces 22 and 23. The third face 22 and the top face 24 are joined by a curved surface 25.

The fourth face 23 and the top face 24 are joined by a curved surface 26.

The first and second faces 20 and 21 have, at the surfaces 25 and 26, a curvilinear profile 27. Preferably, the curved surfaces 25 and 26 and the profile 27 corresponding to them have circular curvature of radius R.

The value of the radius R is between 1.5 mm and 10 mm and, more specifically, is 6 mm.

Advantageously, each hooking element 12 also comprises a plurality of locking teeth 16 located on the sides of the hooking element 12 at the ends 12c, 12d of the hooking element 12 itself.

Since the size of the tabs 15 corresponds to the longitudinal distance between the locking teeth 16 when the hooking elements 12 are raised, the tabs 15 lie between the lock teeth 16. In this configuration, the teeth 16 prevent the hooking elements 12 from accidentally coming out of the respective receiving cavities 13.

The teeth 16 are made as one with the element 12 and for convenience of description are referred to as forming part of the second flat portion 12b that extends horizontally in Figure 3.

The second flat portion 12b comprises the teeth 16. With reference in particular to Figures 3 to 6, it may be observed that the bottom portion 12b of the hooking element 12 does not have sharp edges and that all the flat surfaces are joined by curvilinear surfaces.

The portion 12a has a bottom face 28 and a top face 29 joined to each other at the respective long sides, along their outside profile, by a curved surface 30.

The third and fourth faces 22 and 23 of the top portion 12a are joined to the bottom face 28 of the second portion 12b by curved surfaces 31.

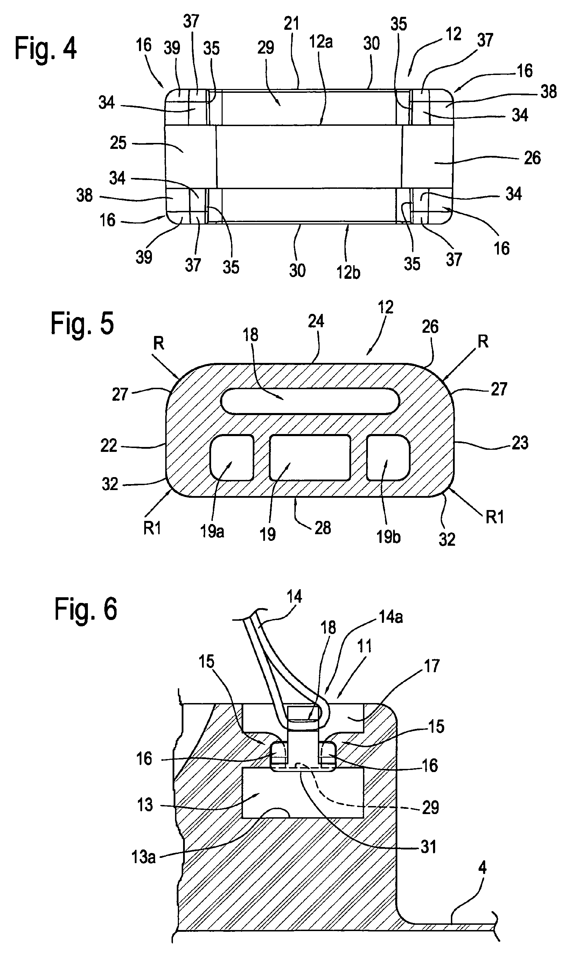

The curved surfaces 31 have a curvilinear profile 32 (Figure 5), preferably circular of radius R1. The radius R1 has a value of between 1 mm and 5 mm or equal to 3 mm in order to facilitate insertion of the element 12 into the cavity 13.

The teeth 16 (Figures 3 and 4) have a first face 33 parallel to the faces 20 and 21 of the first portion 12a.

Each tooth 16 has a second face 34 parallel to the top face 24 of the first portion 12a and to the faces 28 and 29 of the bottom portion 12b.

The description which follows is made, for simplicity, with reference to one tooth 16 only since all the teeth 16 are identical.

The tooth 16 has a third face 35 for engaging the tabs 15 when the elements 12 are raised.

A fourth face 36 parallel to the third face 35 externally delimits the teeth 16 and the hooking element 12.

The face 34 is joined to the face 33 by a curved surface 37.

The face 34 and the face 36 are joined to each other by a curved surface 38.

The face 38 and the face 33 are also joined to each other by a curved surface 39.

The faces 33 and 36 are joined to each other by a curved surface 40.

It is important to observe that the surfaces or faces 30, 31, 33 and 40 are joined by a curved surface 41.

Preferably, all the joining surfaces are either in the form of cylindrical surfaces such as, for example, the surfaces 37, 38, 40, or in the form of spherical surfaces, such as, for example, the surfaces 39, 41.

More specifically, the surfaces 40 have a curvature radius R2 of between 1 mm and 3 mm. Preferably, the radius R2 measures 1.5 mm (Figure 4).

As mentioned, both the receiving portion 10 and the receiving cavities 13 and, in particular, the tabs 15, are made by vacuum thermoforming of the bottom wall 4 of the box-shaped body.

Advantageously, the entire box-shaped body 2 is made by vacuum thermoforming of a laminated sheet of plastic material.

The invention achieves the above mentioned aims and offers important advantages.

Indeed, since the hooking elements are all rounded, it is very easy to insert them into the respective cavities and there is less strain on personnel in charge of assembly.

The hooking elements are equally easy to extract in order to separate the straps from the container when the latter is no longer used.

The absence of sharp edges reduces the risk of breaking the sterile barrier required of the container, in particular at the cavities for the hooking elements.

## Claims

1. A container for sterile medical devices comprising:
- a box-shaped body (2) forming a compartment for containing at least one medical device (D);
- fastening means (11) connected to the box-shaped body (2) to removably secure the medical device (D) inside the containment compartment (3); the fastening means (11) comprising at least one receiving cavity (13) and one hooking element (12), connected to the medical device (D), which can be inserted in the cavity (13), the receiving cavity (13) being made as one with a wall (4) of the box-shaped body (2), the container being **characterized in that** the hooking element (12) has an outer surface with rounded edges.

2. The container according to claim 1, **characterized in that** the hooking clement (12) has an upper portion (12a) and a lower portion (12b) which is transversal to the upper portion (12a), said lower portion having a lower face (28) and an upper face (29) and a curved joining surface (30) between said lower face (28) and upper face (29).

3. The container according to claim 2, **characterized in that** the upper portion (12a) and the lower portion (12b) are joined by a second curved surface (31) which is transversal to the first curved surface (30).

4. The container according to claim 2, **characterized in that** the second curved surface (31) has a circular profile with radius R1.

5. The container according to claim 3, **characterized in that** said radius R1 has a value of between 1 mm and 5 mm or equal to 3 mm.

6. The container according to any of the foregoing claims, **characterized in that** it comprises at least one tooth (16) projecting from the hooking element (12).

7. The container according to claim 5, **characterized in that** the tooth has a prismatic shape and at least one curved joining surface (37, 38, 40) between a pair of adjacent faces of said prism.

8. The container according to claim 5 or 6, **characterized in that** it comprises at least one second joining surface (31) between the tooth (16) and the lower face (28) of the second portion (12b).

9. The container according to claim 5, **characterized in that** the tooth (16) has at least a first face (33), a second face (34), a third face (35) and a fourth face (36) and a first curved joining surface (37) between the first and second faces (33, 34), a second curved joining surface (38) between the second and fourth faces (34, 36) and a third curved joining surface (39) between the first face (33) and the second curved joining surface (38).

10. The container according to claim 8, **characterized in that** the third curved joining surface (39) is formed as a portion of a spherical surface.

11. The container according to claim 8 or 9, **characterized in that** the first and second curved joining surfaces (37, 38) are formed as a portion of a cylindrical surface.

## Patentansprüche

1. Behälter für sterile medizinische Vorrichtungen, umfassend:
- einen kastenförmigen Körper (2), der ein Fach zur Aufnahme mindestens einer medizinischen Vorrichtung (D) bildet,
- Befestigungsmittel (11), die mit dem kastenförmigen Körper (2) verbunden sind, um die medizinische Vorrichtung (D) im Aufnahmefach (3) abnehmbar zu sichern, wobei die Befestigungsmittel (11) mindestens einen aufnehmenden Hohlraum (13) und ein Hakenelement (12), das mit der medizinischen Vorrichtung (D) verbunden ist, die in den Hohlraum (13) eingeführt werden kann, umfassen, wobei der aufnehmende Hohlraum (13) mit einer Wand (4) des kastenförmigen Körpers (2) einstückig ausgebildet ist, wobei der Behälter **dadurch gekennzeichnet ist, dass** das Hakenelement (12) eine äußere Oberfläche mit abgerundeten Ecken aufweist.

2. Behälter nach Anspruch 1, **dadurch gekennzeichnet, dass** das Hakenelement (12) einen oberen Abschnitt (12a) und einen unteren Abschnitt (12b), der transversal zum oberen Abschnitt (12a) ist, aufweist, wobei der untere Abschnitt eine untere Stirnseite (28) und eine obere Stirnseite (29) sowie eine gebogene zusammenführende Oberfläche (30) zwischen der unteren Stirnseite (28) und der oberen Stirnseite (29) aufweist.

3. Behälter nach Anspruch 2, **dadurch gekennzeichnet, dass** der obere Abschnitt (12a) und der untere Abschnitt (12b) durch eine zweite gebogene Oberfläche (31), die transversal zur ersten gebogenen Oberfläche (30) ist, zusammengeführt werden.

4. Behälter nach Anspruch 2, **dadurch gekennzeichnet, dass** die zweite gebogene Oberfläche (31) ein kreisförmiges Profil mit einem Radius R1 aufweist.

5. Behälter nach Anspruch 3, **dadurch gekennzeichnet, dass** der Radius R1 einen Wert zwischen 1 mm und 5 mm oder von 3 mm hat.

6. Behälter nach einem der vorangehenden Ansprüche, **dadurch gekennzeichnet, dass** er mindestens einen Zahn (16) aufweist, der aus dem Hakenelement (12) hervorragt.

7. Behälter nach Anspruch 5, **dadurch gekennzeichnet, dass** der Zahn eine prismatische Form und mindestens eine gebogene zusammenführende Oberfläche (37, 38, 40) zwischen einem Paar benachbarter Stirnseiten des Prismas aufweist.

8. Behälter nach Anspruch 5 oder 6, **dadurch gekennzeichnet, dass** er mindestens eine zweite zusammenführende Oberfläche (31) zwischen dem Zahn (16) und der unteren Stirnseite (28) des zweiten Abschnitts (12b) umfasst.

9. Behälter nach Anspruch 5, **dadurch gekennzeichnet, dass** der Zahn (16) mindestens eine erste Stirnseite (33), eine zweite Stirnseite (34), eine dritte Stirnseite (35) und eine vierte Stirnseite (36) sowie eine erste gebogene zusammenführende Oberfläche (37) zwischen der ersten und zweiten Stirnseite (33, 34), eine zweite gebogene zusammenführende Oberfläche (38) zwischen der zweiten und vierten Stirnseite (33, 34) und eine dritte gebogene zusammenführende Oberfläche (39) zwischen der ersten Stirnseite (33) und der zweiten zusammenführenden Oberfläche (38) aufweist.

10. Behälter nach Anspruch 8, **dadurch gekennzeichnet, dass** die dritte gebogene zusammenführende Oberfläche (39) als ein Abschnitt einer kugelförmigen Oberfläche ausgebildet ist.

11. Behälter nach Anspruch 8 oder 9, **dadurch gekennzeichnet, dass** die erste und zweite gebogene zusammenführende Oberfläche (37, 38) als ein Abschnitt einer zylindrischen Oberfläche ausgebildet sind.

## Revendications

1. Conteneur pour dispositifs médicaux stériles comprenant :
- un corps en forme de boîte (2) définissant un compartiment pour contenir au moins un dispositif médical (D) ;
- des moyens de fixation (11) attachés au corps en forme de boîte (2) pour fixer de façon amovible le dispositif médical (D) à l'intérieur du compartiment de logement (3) ; les moyens de fixation (11) comprenant au moins une cavité de réception (13) et un élément d'accrochage (12) attaché au dispositif médical (D), qui peut être placé dans la cavité (13), la cavité de réception (13) étant solidaire d'une paroi (4) du corps en forme de boîte (2), le conteneur **se caractérisant en ce que** l'élément d'accrochage (12) présente une surface externe à bords arrondis.

2. Conteneur selon la revendication 1, **caractérisé en ce que** l'élément d'accrochage (12) présente une partie supérieure (12a) et une partie inférieure (12b) transversale à la partie supérieure (12a), ladite partie inférieure ayant une face inférieure (28) et une face supérieure (29) et une surface de jonction courbe (30) entre ladite face inférieure (28) et ladite face supérieure (29).

3. Conteneur selon la revendication 2, **caractérisé en ce que** la partie supérieure (12a) et la partie inférieure (12b) sont unies par une seconde surface courbe (31) transversale à la première surface courbe (30).

4. Conteneur selon la revendication 2, **caractérisé en ce que** la seconde surface courbe (31) a un profil circulaire de rayon R1.

5. Conteneur selon la revendication 3, **caractérisé en ce que** ledit rayon R1 a une valeur comprise entre 1 mm et 5 mm ou égale à 3 mm.

6. Conteneur selon l'une quelconque des revendications précédentes, **caractérisé en ce qu'**il comprend au moins une dent (16) en saillie de l'élément d'accrochage (12).

7. Conteneur selon la revendication 5, **caractérisé en ce que** la dent présente une forme prismatique et au moins une surface de jonction courbe (37, 38, 40) entre une paire de faces adjacentes dudit prisme.

8. Conteneur selon les revendications 5 ou 6, **caractérisé en ce qu'**il comprend au moins une seconde surface de jonction (31) entre la dent (16) et la face inférieure (28) de la seconde partie (12b).

9. Conteneur selon la revendication 5, **caractérisé en ce que** la dent (16) a au moins une première face (33), une seconde face (34), une troisième face (35) et une quatrième face (36), et une première surface de jonction courbe (37) entre la première et la seconde faces (33, 34), une seconde surface de jonction courbe (38) entre la seconde et la quatrième faces (34, 36) et une troisième surface de jonction courbe (39) entre la première face (33) et la seconde surface de jonction courbe (38).

10. Conteneur selon la revendication 8, **caractérisé en ce que** la troisième surface de jonction courbe (39) est définie comme une partie d'une surface sphérique.

11. Conteneur selon les revendications 8 ou 9, **caractérisé en ce que** la première et la seconde surfaces de jonction courbes (37, 38) sont définies comme une partie d'une surface cylindrique.
